# EUROPEAN PATENT SPECIFICATION

(11) **EP 3 664 818 B1**
(45) Date of publication and mention of the grant of the patent: **29.11.2023**
(21) Application number: 18890169.8
(22) Date of filing: 06.08.2018
(51) Int. Cl.: A61K 31/045, A61K 31/164, A61K 31/4174, A61K 31/728, A61K 31/4164, A61K 31/415, A61P 11/02, A61K 9/00, C07C 35/12

(54) **NASAL DECONGESTANT COMPOSITIONS COMPRISING MENTHOL, DEXPANTHENOL AND SODIUM HYALURONATE**
NASENABSCHWELLUNGSZUSAMMENSETZUNGEN MIT MENTHOL, DEXPANTHENOL UND NATRIUMHYALURONAT
COMPOSITIONS DE DÉCONGESTIONNANT NASAL COMPRENANT DU MENTHOL, DU DEXPANTHÉNOL ET DU HYALURONATE DE SODIUM

(30) Priority: 10.08.2017 TR 201711880
(43) Date of publication of application: 17.06.2020
(73) Proprietor: Elixir Ilaç Arastirma Ve Gelistirme A.S., 06800 Çankaya/Ankara (TR)
(72) Inventor: YERLIKAYA, Firat, Çankaya/Ankara (TR); AYDIN, Dilara, Çankaya/Ankara (TR); BANOGLU, Erden, Yenimahalle/Ankara (TR); ILERI, Fikret, Yenimahalle/Ankara (TR)
(74) Representative: Bulut, Pinar
(86) International application number: PCT/TR2018/050414
(87) International publication number: WO 2019/125330

(56) References cited:
- WO-A1-2013/097911
- WO-A1-2019/025040
- DE-U1-202014 105 553
- DE-U1-202015 104 761
- US-A- 5 376 365
- US-A- 5 801 199
- US-A1- 2005 164 979
- US-A1- 2011 245 202
- US-A1- 2013 156 868
- Anonymous: "RC-Gel NASAL - Care products R.Cegla Shop", , 1 December 2016 (2016-12-01), XP055710812, Retrieved from the Internet: URL:https://www.cegla-shop.com/en/rc-gel-n asal.html [retrieved on 2020-07-01] & Rc Medizintechnik: "DE Gebrauchsanweisung EN Instructions for use IT Instruzioni per l'uso FR Notice d'utilisation Rc-GEl Nasal", , 1 December 2016 (2016-12-01), XP055710801, Retrieved from the Internet: URL:https://www.cegla-shop.com/media/wysiw yg/GBA_RC-Gel-Nasal_multilingual_124x52_20 16-11_final_Ansicht.pdf [retrieved on 2020-07-01]

## Description

### Field of the Invention

The present disclosure is related to a nasal administration of a pharmaceutical composition in spray form which comprises the combination of xylometazoline, oxymetazoline or a pharmaceutically acceptable salt thereof which is a decongestant effective imidazoline derivative and used in the symptomatic treatment of upper respiratory tract infections and inflammations such as allergic rhinitis and sinusitis, or after the nasal surgery operations, dexpanthenol or its pharmaceutically acceptable salts with moisturizing effect which is used to relieve irritation of the most frequently observed side effects of topical decongestants, and hyaluronic acid or its pharmaceutically acceptable salts.

The invention is related to a pharmaceutical composition which comprises the combination of menthol, which is an effective ingredient in relieving the obstruction, dexpanthenol or its pharmaceutically acceptable salts with moisturizing effect, and hyaluronic acid or its pharmaceutically acceptable salts.

### Background of the Invention

Xylometazoline and oxymetazoline are imidazoline-derived sympathomimetic agents that have widespread use as topical decongestants. These imidazoline-derived compounds, which are used as nasal decongestants, directly stimulate α-adrenoceptors and alleviate the obstruction by decongestion effect by narrowing the nasal vessels, especially sinusoidal venules in the nasal mucosa. These compounds show a greater affinity for the α-adrenoceptors than for adrenaline and noradrenaline, and it is argued that they selectively stimulate only α2 receptors.Nasal mucosal α2 receptors are found more intensely than α1 adrenoceptors, and α2 receptors influence nasal sinusoid venules while α1 receptors affect venous and arterial nerves surrounding nasal mucosa. Thus, several sources argue that selective α2-adrenoceptor agonists such as xylometazoline and oxymetazoline are a better alternative to non-selective agents in order to provide effective decongestion in the nasal mucosa region in which α2-adrenoceptors are intensively present, while also providing a safer treatment by reducing the possibility of systemic side effects. The decongestant activity of xylometazoline and oxymetazoline is rapid and long-lasting and is commonly used in the form of hydrochloric acid salt in nasal application composition as there is no known systemic toxicity.The chemical name of the xylometazoline hydrochloride is 2-[(4-tert-butyl-butyl-2,6-dimethylphenyl)methyl]-4,5-dihydro-1H-imidazole hydrochloride and its chemical structure is as shown in Figure 1.The chemical name of oxymetazoline hydrochloride is 6-tert-butyl-3-(4,5-dihydro-1H-imidazol-2-ylmethyl)-2,4-dimethylphenol hydrochloride and its chemical structure is as shown in Figure 2.

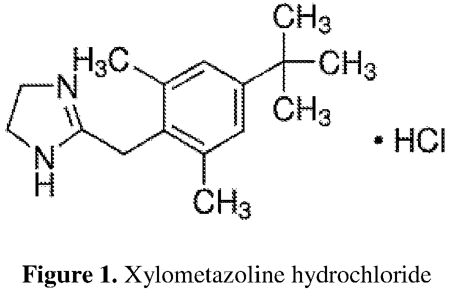

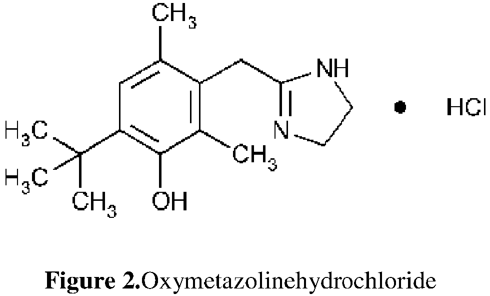

Xylometazoline hydrochloride is presented in the form of nasal spraysand nasal drops in solution form, with a strength of 0.1% (w/v) in adults and 0.05% (w/v) in children. Oxymetazoline hydrochloride is presented in nasal sprays and nasal drops in the form of solution, with a strength of 0.05% (w/v) in adults and 0.025% (w/v) in children.

Menthol is commonly used to relieve symptoms of upper respiratory tract disorders such as bronchitis and sinusitis. Menthol is extracted from various mint oil mixtures. It is an alcohol in the cyclic terpene group and have three asymmetric carbons in the cyclohexane structure. The aromatic volatile component, menthol, has four pairs of optical isomers. Although the mechanism of action is not fully understood, it is argued that topical application narrows blood vessel and provides relief following analgesic effect in a mild manner. It exerts its effects especially over the thermoreceptors found on the skin and in the mucosa. The mechanism of action for eliminating occlusion is explained by the opening of calcium channels via TRPM8 thermoreceptors inducing sensory nerves. In this way, it provides a refreshing response to the area of application. The interaction of menthol isomers with TRPM8 thermoreceptors differs from each other. The refreshing activity of L-menthol was found to be higher than the other isomers. As a result of its use at high concentrations, burning, pain and mucociliary functions, and consequently nasal congestion can be observed.

It has been reported that a high dose of 2-5% (w/v) topical application causes a similar effect to irritation and local anesthetic effect. No irritant effect for nasal administration reported in 0.1% (w/v) concentration. The chemical name of the menthol is (1R, 2S, 5R)-5-methyl-2-propan-2-yl cyclohexan-1-ol and its chemical structure is as shown in Figure 3.

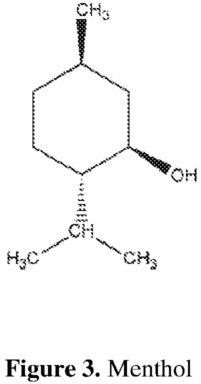

Menthol is usually presented in the form of lozenges, solution for inhalation, nasal spray, mouthwash, syrup, topical cream and ointment in market preparations.

The most common side effects of decongestant drugs are dryness; and the rebound congestion with long-lasting and frequent use. Systemic side effects, possible undesirable situations such as mucous membrane irritation and recurrent congestion requires restricting the usage period and frequency of drug. Generally, nasal decongestants, which should be used for a maximum of 3 to 5 days, can cause rebound congestion, i.e. mucous membrane recurrent congestion and edema in the case of longer usage.The presence of obstruction in the nasal decongestant caused by rebound congestion may cause the nasal decongestant to be used more often and for a longer period of time in order to relieve the patient's obstruction. Therefore, a feeling of burning in the throat due to misuse of the patient's nasal decongestant can be observed, changes observed in the mucociliary system in the nasal mucosa, the heat distribution in the resultant nasal airway becomes unbalanced, the water holding capacity of the mucus and the ability to heat and humidify air are reduced, and cases such as dryness and crusting can be observed in patients. The nasal mucus, which suffers from loss of moisture content and irritation, cannot perform the function of protection and filtration and causes respiratory tract infections. Although the mentioned adverse effects are seen in all topical decongestants, xylometazoline and oxymetazoline are the compounds with least systemic side effects between these drugs and are recommended for effective decongestant treatment when physiological saline solutions are inadequate. While patients have access to effective and safe treatment with decongestant drugs, it is necessary to reduce the complaints related to the patients. A variety of active substances and excipients are included in the compositionsin order to eliminate the undesirable effects such as dryness and mucosal irritation, which can be observed with the use of topical decongestant, and to effectively treat the obstruction. Pentanoic acid derivatives and hyaluronic acid or physiological salt forms/derivatives are well-known agents that protect the nasal epithelium against damage creating a protective film layer in the nasal mucosa and restoring the moisture lost. Both compounds which are toxicologically and immunologically reliable are used in topical medicines.

Panthenol compound is also known as pantothenol and pantothenyl alcohol in the structure of (R, S)-2,4-dihydroxy-N- (3-hydroxypropyl) -3,3-dimethylbutyramide. Panthenol is a pantothenic acid analog. Since panthenic acid is not stable in topical formulations, it is usually present in formulations in its panthenol or dexpanthenol forms. Also known as provitamin B5, dexpanthenol is a panthenolic acid alcohol derivative and precursor material. Panthenol; which is dexpanthenol or racemate, is used topically at an amount of 2% (w/v) or 5% (w/v). It has been used for many years in topical formulations to acquire the epithelial tissue function and to accelerate wound healing. There are a few studies showing that dexpanthenol increases the hydration of the stratum corneum layer and decreases trans-epidermal water loss. It has been shown that preparations containing dexpanthenol have a favorable result in both spray and ointment formulations and provide prominently moisturization after intranasal application. The chemical name of dexpanthenol is (2R)-2,4-dihydroxy-N-(3-hydroxypropyl)-3,3-dimethylbutanamide, and its chemical structure is as shown in Figure 4.

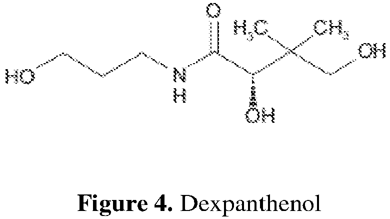

Another moisturizing agent known in the state-of-the-art is hyaluronic acid or pharmaceutically acceptable salt forms. Hyaluronic acid, a compound of the glycosaminoglycan group, is a biomolecule of polyanionic polysaccharide. It is used in the treatment of osteoarthritis by transient replacement and support of synovial fluid and in the treatment of osteoarthritis-related pain and in the reduction of pain or the relief of joint mobility. It is used for wound healing, especially for the healing and regeneration of surgical wounds. It is also used as mucoadhesive polymer in ophthalmic, nasal and parenteral drug delivery systems. It takes place in form of sodium hyaluronate which is hyaluronic acid sodium salt, physiological stabilized form, in the topical formulation improves of the protective function of the mucosal membrane and provides adequate hydration, and preservatives of the epithelial integrity. The chemical name of sodium hyaluronate is sodium 6-[3-acetamido-2-[6-[3-acetamido-2,5-dihydroxy-6- (hydroxymethyl)oxan-4-yl]oxy-2-carboxy-4,5-dihydroxyoxan-3-yl]oxy-5-hydroxy-6-(hydroxymethyl)oxan-4-yl] oxy-3,4,5-trihydroxyoxane-2-carboxylic acid as shown in Figure 5.

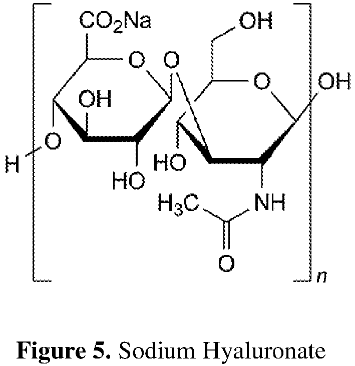

Sodium hyaluronate is presented usually in the form of intraarticular injectable solutions, spray for intraoral lesion, intraocular injection solutions, vaginal ovule, eye drops and nasal spray forms in market preparations.

Hyaluronic acid and xylometazoline hydrochloride are available in the market as Rhinostop^{®} IBSA (Italy). Due to the presence of hyaluronic acid, xylometazoline monotherapy is effective at half dose of the therapeutic dose in preventing irritation which can occur as a result of decongestant use. Common cold and upper respiratory tract infections, seasonal allergic rhinitis are the most common pathologies in winter. Thus, topical decongestants in the symptomatic treatment of respiratory tract disorders is a common drug group. However, the adverse effects of the decongestant imidazoline derivatives and other antimucosal components of the drug on long-term and frequent use of the nasal health, the success of the treatment is overshadowed in a significant way. The use of drugs containing active ingredients known to have mucosal and epithelial protective, moisturizing and healing functions is suggested to overcome these undesirable effects. However, since most of these undesirable effects are due to decongestant use, the use of topical decongestants or nasal moisturizers alone in such disorders is not sufficient to protect the health of the nose. The administration of each drug separately is challenging in terms of patient compliance. Because patients generally do not prefer to take many medicines, it is especially difficult to obtain patient compliance in special populations such as elderly and children. In addition to this general situation, due to the high volume it is not possible to keep more than one drug in a liquid form applied locally in the nasal cavity,some liquid containing active ingredients are removed by drainage from the nasal route. Thus, there is a need for the development of pharmaceutical compositions containing a combination of the active ingredients with the indicated indications for symptomatic treatment. Thus, patient complaints will be reduced due to the use of decongestant and patient compliance will be increased.

There are some difficulties in preparing pharmaceutical compositions containing the active ingredients in combination. These include the incompatibility of the active ingredients with each other and the difficulties experienced when the active substances in different physicochemical structures are formulated together. When an active ingredient is used in combination with other active ingredients, the chemical stability may be affected and the difficulties in developing formulation may be observed. Although they are expected to show their own therapeutic effects in a combination, it is known that the drugs can not always be combined even in the same therapeutic area and even the drugs used together for the treatment of the same disease. Scientific literature are full of examples in which the compounds from different classes used to treat the same indications that cannot be combined in reliable and effective dosage forms, thus resulting in incompatible drug combinations. The reasons for this unexpected incompetence are very different; but mainly due to chemical or physical incompatibility, increased side effects, undesired drug interactions or new side effects. Nasic^{®} (Germany) is a combination of dexpanthenol and xylometazoline hydrochloride. Xylometazoline has been shown to provide a significant superiority over monotherapy. Hysan^{®} pflegespray (Germany) is a combination of dexpanthenol and sodium hyaluronate. These preparations contain the active ingredients in dual combinations in thestate of the art to provide a synergistic effect for the protection and improvement of intranasal health.

TR 2010/01327 (Klosterfrau) comprises a formulation comprising 0.001-1% xylometazoline, 0.01-5% hyaluronic acid and 0.01-15% panthenol (dexpanthenol) by weight. In the state of art mentioned in the description, reference is made to the earlier application EP0773022 (Klosterfrau). This application is related to a formulation containing xylometazoline and dexpanthenol.

EP 2822537 B1 (Klosterfrau) is related to a formulation containing panthenol (dexpanthenol) and/or pantothenic acid and xylometazoline HCl for nasal rhinitis treatment. The formulation is given in addition may comprise hyaluronic acid.

EP 2766008 B1 (Klosterfrau) is related to a formulation comprising xylometazoline, dexpanthenol and benzalkonium chloride. It is stated in the claims that hyaluronic acid may be added to the formulation.

RC-Gel NASAL - Care products R.Cegla Shop, https://www.cegla-shop.com/en/rc-gel-nasal.html, discloses a nasal composition comprising menthol, dexpanthenol and sodium hyaluronate.

### Detailed Description of the Invention

The invention is defined by the claims.

In a non-claimed aspect, the present disclosure is related to a nasal pharmaceutical composition which comprises a decongestant compound selected from xylometazoline, oxymetazoline, menthol or a pharmaceutically acceptable salt thereof; dexpanthenol or a pharmaceutically acceptable salt thereof and hyaluronic acid or a pharmaceutically acceptable salt thereof.

The disclosure aims to improve the treatment efficacy with a nasally administrable spray formulation which comprises a decongestant like xylometazoline or oxymetazoline or their pharmaceutically acceptable salts, which are decongestant effective imidazoline derivative compounds, used in the symptomatic treatment of upper respiratory tract infections and inflammations such as allergic rhinitis and sinusitis or in the symptomatic treatment of these occlusions following nasal surgical operations, dexpanthenol or its pharmaceutically acceptable salts with moisturizing effect, which is used to relieve irritation of the most frequently observed side effects of topical decongestants, and hyaluronic acid or its pharmaceutically acceptable salts.

By adding these active ingredients into a nasal decongestant formulation, it is aimed that prevention of the nasal mucosa dryness, which is the most frequently observed adverse effect of xylometazoline and oxymetazoline, shortening the healing time of the patients and providing better tolerance. Thus, when the desired therapeutic efficacy is achieved with the active ingredients of the combination, possible side effects are avoided and patient compliance increased by making it possible to treat more than one symptom at the same time. In the state of the art, it appears that the combination pharmaceutical products for the symptomatic treatment provide overall benefits compared to pharmaceutical products containing the active substance alone.

However, some disadvantages observed in the state of art and the lack of available solutions have led to the need to develop a new pharmaceutical composition that is used to relieve symptoms related to diseases such as colds, upper respiratory tract infections and seasonal allergic rhinitis.

The present disclosure relates to a novel composition comprising three active ingredients prepared for using in the symptomatic treatment of colds, upper respiratory tract infections and seasonal allergic rhinitis which fulfill the above requirements, with removing some of the existing disadvantages of compositions and providing some additional advantages. Therefore, in a non-claimed aspect, the present disclosure provides a novel pharmaceutical composition comprising a combination of xylometazoline, oxymetazoline or a pharmaceutically acceptable salt thereof which are imidazoline derivatives, dexpanthenol, hyaluronic acid or apharmaceutically acceptable salt.

The present invention provides a novel pharmaceutical composition comprising menthol which is an effective agent, and dexpanthenol and hyaluronic acid or pharmaceutically acceptable salts thereof for the relief of symptoms associated with upper respiratory tract infections, colds and allergic rhinitis. The present invention is a pharmaceutical composition comprising an active ingredient which provides relief to the nasal congestion and a well-known active ingredient which protect the nasal epithelium against damage creating a protective film layer in the nasal mucosa and restoring the moisture lost. This composition relates to suitable pharmaceutical ingredients in terms of stability and these ingredients are compatible with each other.

The combination of panthenol or its esters and/or their physiologically harmless salts and decongestant effective compounds in the state of art has shown that decongestant has sympathomimetic activity as well as preventing mucosal irritation and is effective against nasal dryness. In this regard, the active substance that promotes the protection of intranasal health in the present invention is panthenol. When the effect of panthenol on long-term transdermal water loss and deep barrier function is examined in the literature, it has been observed that formulations containing 1% to 5% panthenol significantly reduce transdermal water loss and provide adequate hydration. Panthenol has been shown to reduce trans-epidermal water loss through its film-forming structure and protective properties, as well as its stratum corneum moisturizing effect. In the present invention, dexpanthenol, S-enantiomer of panthenol is preferred. Adding dexpanthenol into the pharmaceutical composition of the invention will prevent the undesirable effects such as dryness of the nasal mucosa, irritation and inflammation which are associated with the use of nasal decongestant and provide effective treatment to accelerate recovery in damaged tissue with adequate hydration. In this context, dexpanthenol is included in the composition to provide therapeutic benefit in relieving symptoms related to diseases such as upper respiratory tract infections, colds and allergic rhinitis.

The combination of hyaluronic acid or its pharmaceutically acceptable salts with decongestant active substances in the state of the art has shown that decongestant has sympathomimetic activity as well aspreventing mucosal irritation and efficacy against nasal dryness. In this regard, another active ingredient in the present invention that promotes intranasal health is hyaluronic acid. Hyaluronic acid components are obtained by different methods (such as purification, different solvents in isolation processes) from different sources (bacterial fermentation, chemo-enzymatic method, isolation from animal tissue etc.). The biological function of hyaluronic acid determines the molecular weight, intrinsic viscosity and physicochemical properties, and water retention capacity is very high depending on the molecular weight. In the present invention, the physiological stabilizing form of hyaluronic acid sodium salt, in other words sodium hyaluronate, is preferred. Incorporation of sodium hyaluronate into the pharmaceutical composition of the invention will prevent of undesirable effects such as dryness of the nasal mucosa, irritation and inflammation which are associated with the use of nasal decongestant and provide effective treatment to accelerate recovery in damaged tissue with adequate hydration. Aqueous sodium hyaluronate solutions exhibit non-Newtonian flow properties and are characterized by decreasing viscosity with increasing shear rate. It is known that sodium hyaluronate increases wetting capability of the nose, spreads widely over a wide range depending on its concentration and molecular weight. High molecular weight hyaluronic acid exhibits high viscoelastic properties at low concentrations. It is argued that decongestant is retained better and longer on the nasal mucosal membrane with hygroscopic structure of hyaluronic acid, water binding capacity, viscoelastic properties and that the disposal of mucosal membranes without their effect is also inhibited in this way. With the present invention it has been found that xylometazoline or oxymetazoline hydrochloride has no stability problems in combination with dexpanthenol and sodium hyaluronate and that the active ingredients are not incompatible with each other. This combination has also resulted in an effective and novel composition for relieving symptoms related to diseases such as upper respiratory tract infections, colds and allergic rhinitis. Therapeutic benefit will be achieved by incorporating dexpanthenol and sodium hyaluronate, a well-known substance that protects the nasal epithelium against damage creating a protective film layer in the nasal mucosa and restoring the moisture lost.

The pharmaceutical composition to be developed in the context of the invention contains menthol,which is an effective agent for eliminating the occlusion here, instead of the imidazoline derivative topical decongestants. In this case, it is aimed to provide benefit with a pharmaceutical combination of dexpanthenol and hyaluronic acid, or a pharmaceutically acceptable salt thereof, which is effective in relieving symptoms such as obstruction that moisturizes the nose without undesirable effects, such as nasal epithelial damage and impaired intranasal health, which are associated with the use of symptomatic drugs.It has been found by the present invention that there is no stability problem in combination with menthol, dexpanthenol and sodium hyaluronate, and the active ingredients are not incompatible with one another. This combination has also resulted in an effective and novel composition for relieving symptoms related to diseases such as upper respiratory tract infections, colds and allergic rhinitis. Therapeutic benefit will be achieved by incorporating dexpanthenol and sodium hyaluronate, a well-known substance that that protects the nasal epithelium against damage creating a protective film layer in the nasal mucosa and restoring the moisture lost. The menthol in the context of the invention is L-menthol.

The molecular weight of sodium hyaluronate for humectant effect in topical preparations according to the present invention is 800-1700 kDa. Sodium hyaluronate is used in the composition at an amount from 0.01 to 5% by weight.

In a non-claimed aspect, the disclosure comprising xylometazoline hydrochloride at an amount from 0.05 to 0.1% by weight, dexpanthenol at an amount from 2 to 5% by weight, and sodium hyaluronate at an amount from 0.01 to 5% by weight for each dose unit to be administered.

The disclosure comprising xylometazoline hydrochloride at an amount of 0.025 to 0.05% by weight, dexpanthenol at an amount from 2 to 5% by weight, and sodium hyaluronate at an amount from 0.01 to 5% by weight for each dose unit to be administered.

The invention is a composition comprising from menthol at an amount from 0.002 to 0.2% by weight, dexpanthenol at an amount from 2 to 5% by weight, and sodium hyaluronate at an amount from 0.01 to 5% by weight for each dose unit to be administered.

The molecular weight of the sodium hyaluronate to be used in the context of the present invention is in the range of 800 to 1700 kDa.

The combination is applied topically to the intranasal route of application. The pharmaceutical composition according to the invention is in solution or suspension form as drop or spray.

In the present invention, the formulation contains, in addition to the three active ingredients, at least one pharmaceutically acceptable excipient.

Nasal spray formulations contemplated to be developed within the scope of the invention may additionally contain compounds of the polyol group due to both the moisturizing effect and the growth inhibitory effect of certain upper respiratory tract infections.

The polyols improve the stability of the formulation due to their low water activity. For this purpose, polyols such as glycerin and sorbitol can be used.

In the context of the present invention, another feature of the pharmaceutical composition is involving sodium chloride or sea salt. Instead, or in addition, physiological salts, buffers or other ionic, non-ionic physiologically acceptable substances, mineral substances may be used.

The use of sodium chloride or sea salt is quite common in the state of the art.

The pharmaceutical compositions that were developed according to the invention are prepared in the range of pH 4.0-7.4 in terms of their physiological acceptability. More preferably, the invention is preferably not below pH 6.5. Preferred pH modifying agents within the scope of the present invention are sodium hydroxide, sodium phosphate, sodium citrate or pharmaceutically acceptable buffer systems. Phosphate buffer systems are the most common use of buffering components included in the formulation according to the pKa value of the drug in the state of the art.

In the context of the invention, solvents (alcohol, propylene glycol etc.), preservatives (benzalkonium chloride, sorbic acid, phenyl alcohol, potassium sorbate, propyl paraben, methyl paraben, isopropyl alcohol, sodium benzoate etc.), antioxidants (ascorbic acid, butyl hydroxy toluene, butyl hydroxyanisole, vitamin A, vitamin K, carotene etc.) surfactants (polysorbate, polyoxyl stearate, sorbitan esters), humectants (such as glycerol, sorbitol, Aloe Vera, menthol, camphor, etc.) can be used. The number and type of adjuvants which may be used in this connection are not limited to those described herein.

Examples of this invention are given in Tables 1-3.

**Table 1. Nasal spray formulations comprising xylometazoline hydrochloride (not part of the present invention).**

| **Active Substance/Excipients** | **Pediatric Formulation** | **Adult Formulation** |
|---|---|---|
| Xylometazoline hydrochloride | 5 mg | 10 mg |
| Dexpanthenol | 100 mg | 200 mg |
| Sodium hyaluronate | 2 mg | 4 mg |
| Sorbitol | 100 mg | 100 mg |
| Glycerin | 100 mg | 100 mg |
| Sodium chloride | 90 mg | 90 mg |
| Benzalkonium chloride | 0.1 mg | 0.1 mg |
| Water | *qs* 10 mL | *qs* 10 mL |

**Table 2. Nasal spray formulations comprising oxymetazoline hydrochloride (not part of the present invention).**

| **Active Substance/Excipients** | **Pediatric Formulation** | **Adult Formulation** |
|---|---|---|
| Oxymethazoline hydrochloride | 2.5 mg | 5 mg |
| Dexpanthenol | 100 mg | 200 mg |
| Sodium hyaluronate | 2 mg | 4 mg |
| Sorbitol | 100 mg | 100 mg |
| Glycerin | 100 mg | 100 mg |
| Sodium chloride | 90 mg | 90 mg |
| Benzalkonium chloride | 0.1 mg | 0.1 mg |
| Water | *qs* 10 mL | *qs* 10 mL |

**Table 3. Nasal spray formulations comprising menthol.**

| **Active Substance/Excipients** | **Pediatric Formulation** | **Adult Formulation** |
|---|---|---|
| Menthol | 10 mg | 20 mg |
| Dexpanthenol | 100 mg | 200 mg |
| Sodium hyaluronate | 2 mg | 4 mg |
| Sorbitol | 100 mg | 100 mg |
| Glycerin | 100 mg | 100 mg |
| Sodium chloride | 90 mg | 90 mg |
| Benzalkonium chloride | 0.1 mg | 0.1 mg |
| Water | *qs 10* mL | *qs 10* mL |

## Claims

1. A nasal pharmaceutical composition in solution or suspension form as drop or spray comprising,
- a decongestant effective compound which is L-menthol,
- dexpanthenol, and
- sodium hyaluronate,
wherein the molecular weight of sodium hyaluronate is 800-1700 kDa and the amount of sodium hyaluronate is from 0.01 to 5% by weight, and
the amount of L-menthol is from 0.002 to 0.2% by weight and the amount of dexpanthenol is from 2 to 5% by weight.

## Patentansprüche

1. Nasale pharmazeutische Zusammensetzung in Lösungs- oder Suspensionsform als Tropfen oder Spray, umfassend
- eine abschwellend wirksame Verbindung, die L-Menthol ist,
- Dexpanthenol, und
- Natriumhyaluronat,
wobei das Molekulargewicht von Natriumhyaluronat 800-1700 kDa beträgt und die Menge an Natriumhyaluronat 0,01 bis 5 Gew.-% beträgt, und
die Menge an L-Menthol von 0,002 bis 0,2 Gew.-% und die Menge an Dexpanthenol von 2 bis 5 Gew.-% beträgt.

## Revendications

1. Composition pharmaceutique nasale sous forme de solution ou de suspension, en goutte ou en spray, comprenant,
- un composé décongestionnant efficace qui est le L-menthol,
- dexpanthénol, et
- hyaluronate de sodium,
dans lequel le poids moléculaire de l'hyaluronate de sodium est compris entre 800-1700 kDa et la quantité d'hyaluronate de sodium est comprise entre 0.01 à 5% en poids, et
la quantité de L-menthol est comprise entre 0.002 à 0.2 % en poids et la quantité de dexpanthénol est comprise entre 2 à 5% en poids.
